Europäisches Patentamt

European Patent Office   (11) Publication number:   **0 077 956**

Office européen des brevets   **B1**

(12)   **EUROPEAN PATENT SPECIFICATION**

(45) Date of publication of patent specification: **03.07.85**   (51) Int. Cl.⁴: **B 01 J 13/02, A 61 K 9/52**

(21) Application number: **82109335.8**

(22) Date of filing: **08.10.82**

(54) Enteric microcapsules and process for the preparation thereof.

(30) Priority: **15.10.81 JP 165149/81**

(43) Date of publication of application:
**04.05.83 Bulletin 83/18**

(45) Publication of the grant of the patent:
**03.07.85 Bulletin 85/27**

(84) Designated Contracting States:
**AT BE CH DE FR GB IT LI LU NL SE**

(56) References cited:
**DE-A-2 721 603**
**GB-A-1 469 133**
**GB-A-2 009 698**
**US-A-3 247 066**
**US-A-3 748 277**
**US-A-3 939 259**
**US-A-3 960 757**
**US-A-4 123 382**

(73) Proprietor: **Tanabe Seiyaku Co., Ltd.**
**No. 21 Dosho-machi 3-chome Higashi-ku**
**Osaka-shi Osaka-fu (JP)**

(72) Inventor: **Samejima, Masayoshi**
**No. 2515-23, Oaza-Aomadani**
**Minoh-shi Osaka-fu (JP)**
Inventor: **Hirata, Goichi**
**No. 13-9, Nishiyama Adachi Yawata-shi**
**Kyoto-fu (JP)**
Inventor: **Koida, Yoshiyuki**
**No. 36-6, Amanogahara-cho 2-chome**
**Katano-shi Osaka-fu (JP)**
Inventor: **Kobayashi, Yoshinori**
**No. 4-16, Kita-Sakurazuka 2-chome**
**Toyonaka-shi Osaka-fu (JP)**
Inventor: **Kida, Akira**
**No. 4, Minamibefu 6-chome**
**Settsu-shi Osaka-fu (JP)**

(74) Representative: **Hansen, Bernd, Dr.rer.nat. et al**
**Hoffmann, Eitle & Partner Patentanwälte**
**Arabellastrasse 4**
**D-8000 München 81 (DE)**

Courier Press, Leamington Spa, England.

**Description**

The present invention relates to a process for the preparation of enteric microcapsules containing an active compound as a core material, the coating walls of which consist essentially of ethylcellulose and an enteric polymer material.

It is known to control the release of a core material in microcapsules by thickening the coating walls of microcapsules or by forming compact coating walls and thereby decreasing the permeability thereof (cf. Tamotsu Kondo and Masumi Koishi; "Microcapsules, Process for the Preparation thereof, Their Properties and Applications", issued by Sankyo Shuppan, 1977). According to these known methods, however, while the release of core material is well controlled, the release of core material is also inhibited even when the core material should be released and hence the desired activities of the main active compounds are occasionally not obtained. Particularly, in case of pharmaceutical compounds, they are usually microencapsulated with ethylcellulose in order to mask unpleasant odor or taste thereof, but in some cases, such microcapsules show retarded release of the active ingredient in intestinal tract.

GB—A—1 469 133 discloses a method for preparing ethylcellulose microcapsules having walls containing an enteric polymer material.

An object of the present invention is to provide a process for the preparation of enteric microcapsules being capable of releasing easily the active component (core material) in intestinal tract while protecting the core material effectively in stomach. This object and advantages of the present invention will be apparent to persons skilled in the art from the following description.

The enteric microcapsules obtained by the present invention (i.e. enteric microcapsules containing an active compound as a core material, the coating walls of which consist essentially of ethylcellulose and an enteric polymer material) can be prepared by the steps of:

(a) ethylcellulose is dissolved in a solvent at a temperature of about 75—80°C,

(b) particles of a core material are disposed in the solution thus obtained,

(c) the dispersion is cooled until coating walls having a viscosity of 0.01 to 5 Pa . s are formed on and around particles of the core material,

(d) an enteric polymer material is added to the dispersion,

(e) the dispersion containing the enteric polymer material is further cooled to a temperature not higher than 40°C until the resultant embryonic microcapsules shrink and become solid by solvent loss from the coating walls, and

(f) the thus-formed microcapsules are recovered therefrom.

The core material used in the present invention includes pharmaceutical compounds and foodstuffs which may be in the form of a solid, gel or semi-solid. The particle size of the core material is not critical but is usually in the range of about 30 to 1,000 μm, preferably about 50 to 500 μm.

Ethylcellulose used for forming microcapsule coating walls on and around the particles of the core material has preferably an ethoxy content of about 46.5 to 55 W/W % and a viscosity of about 3 to 500 mPa . s (the viscosity of ethylcellulose is measured in a 5 W/W % solution in toluene-ethanol (4:1) at 25°C). The ethylcellulose is usually used in an amount of about 0.01 to 10 grams per gram of the core material.

The enteric polymer material to be incorporated into the ethylcellulose coating walls includes (i) an organic dicarboxylic acid ester of a hydroxyalkyl alkylcellulose or cellulose acetate; (ii) a carboxyalkyl alkylcellulose; (iii) a copolymer of an alkenylcarboxylic acid and an alkyl ester of an alkenylcarboxylic acid; (iv) a copolymer of an alkenylcarboxylic acid and two alkyl esters of an alkenylcarboxylic acid, (v) zein and (vi) shellac. Suitable examples of the organic dicarboxylic acid esters of a hydroxyalkyl alkylcellulose or cellulose acetate (i) are phthalic acid esters of a hydroxyalkyl alkylcellulose or cellulose acetate, such as hydroxyethyl ethylcellulose phthalate, hydroxypropyl methylcellulose phthalate, cellulose acetate phthalate, and other organic dicarboxylic esters such as cellulose acetate succinate, cellulose acetate maleate, or the like. Suitable examples of the carboxyalkyl alkylcellulose (ii) are carboxymethyl methylcellulose and carboxymethyl ethylcellulose. Suitable examples of the copolymer of an alkenylcarboxylic acid and an alkyl ester of an alkenylcarboxylic acid (iii) are copolymers of methacrylic acid and an alkyl acrylate or methacrylate, such as a copolymer of methacrylic acid and methyl acrylate, a copolymer of methacrylic acid and ethyl acrylate, copolymer of methacrylic acid and methyl methacrylate. Suitable examples of the copolymer of an alkenylcarboxylic acid and two alkyl esters of an alkenylcarboxylic acid (iv) are copolymers of methacrylic acid, an alkyl acrylate and an alkyl methacrylate, such as a copolymer of methacrylic acid, methyl acrylate and methyl methacrylate, a copolymer of methacrylic acid, methyl acrylate and octyl acrylate.

These enteric polymer materials are preferably in the form of a finely-divided particle, particularly having a particle size of about 300 μm or less, more particularly a particle size of 0.1 to 300 μm. The enteric polymer materials are preferably used in an amount of at least about 0.01 gram, more preferably about 0.05 to 20 grams per gram of the coating wall-forming material (ethylcellulose).

When a water-swellable polymer material is incorporated into the core material in microcapsules wherein an enteric polymer material is incorporated in the coating walls, the release of the active compound is more promoted.

Such a water-swellable polymer material is a material which shows at least 1.2 times increase in weight by immersing it in water at 37°C. The water-swellable polymer material includes agar-agar, pectinic acid, alginic acid, cellulose, starch, a carboxyalkyl cellulose (e.g. carboxymethyl cellulose) or its calcium salt, copolymers of divinylbenzene and an alkenylcarboxylic acid (e.g. a copolymer of divinylbenzene and acrylic acid, a copolymer of divinylbenzene and methacrylic acid), a cross-linked gum arabic (e.g. gum arabic cross-linked with epichlorohydrin), a cross-linked dextran (e.g. dextran cross-linked with epichlorohydrin), and a cross-linked polyalkenylcarboxylic acid (e.g. self-crosslinked polyacrylic acid, self-crosslinked methacrylic acid).

These water-swellable polymer materials are preferably in the form of a finely-divided particle, particularly having a particle size of about 300 µm or less, more particularly a particle size of 0.1 to 300 µm. Suitable amount of the water-swellable polymer material to be incorporated into the core material is about 1 to 99 w/w %, especially about 5 to 90 w/w %.

In the preparation of the microcapsules of the present invention, an ethylcellulose dispersion containing a core material is firstly prepared by dispersing a core material into a solution containing ethylcellulose as the wall-forming material. In case of ethylcellulose microcapsules containing a water-swellable polymer material in the core material, it is preferable to previously prepare a granule of a mixture of a core material and a water-swellable polymer material, and the core material containing a water-swellable polymer material is dispersed into a solution of ethylcellulose.

The solvent for dissolving ethylcellulose is a substance which can dissolve ethylcellulose with heating (i.e. at a temperature of about 75 to 80°C) but does not dissolve under cooling and further does not dissolve both of the core material and the water-swellable polymer material. Suitable examples of the solvent are cyclohexane, a mixture of cyclohexane and n-hexane, or the like, among which cyclohexane is particularly suitable. Ethylcellulose is dissolved in such a solvent at a temperature of about 75 to 80°C. The ethylcellulose solution thus obtained has preferably a concentration of ethylcellulose of about 0.1 to 10 W/W %, more preferably about 1 to 5 W/W %. The dispersion of a core material or a core material containing a water-swellable polymer material into an ethylcellulose solution is preferably carried out with stirring at a temperature of about 75 to 80°C. The granule of a mixture of a core material and a water-swellable polymer material can be prepared by a conventional method such as wet-granulation method or dry-granulation method, and the particle size of the granule is not critical but is usually in the range of about 30 to 1,000 µm, preferably 50 to 500 µm.

The phase-separation of ethylcellulose from the dispersion of a core material or a core material containing water-swellable polymer material is carried out in the presence or absence of a phase-separation-inducing agent, i.e. by either coacervation or flocculation, and optionally in the presence of a wall-forming auxiliary and/or a surfactant.

The phase-separation-inducing agent includes polyethylene, butyl rubber, polyisobutylene, and polybutadiene. The wall-forming auxiliary includes dimethyl polysiloxane, methylphenyl polysiloxane, diphenyl polysiloxane, and polystyrene-polydimethyl polysiloxane block copolymer. The surfactant includes an ester of $C_{12-18}$ fatty acid with sorbitan (e.g. sorbitan monolaurate, sorbitan sesquilaurate, sorbitan trilaurate, sorbitan monoleate), an ester of $C_{8-18}$ fatty acid with glycerin (e.g. glycerin monocaprylate, glycerin monolaurate, glycerin monooleate), a phospholipid (e.g. soybean phospholipids, egg-yolk phospholipids), calcium stearyl lactate, an ester of $C_{8-18}$ fatty acid with propylene glycol (e.g. propylene glycol monocaprylate, propylene glycol monostearate), and an ester of $C_{12-18}$ fatty acid with sucrose (e.g. sucrose mono-, di- or tristearate). These additives may be added to the ethylcellulose solution prior to dispersing the core material in said solution. The additives are used in a concentration of about 0.01 to 10 W/V % (phase-separation-inducing agent), about 0.01 to 10 W/V % (wall-forming auxiliary), and about 0.001 to 10 W/V % (surfactant), respectively.

The phase-separation of ethylcellulose is preferably carried out by cooling the dispersion at a rate of about 0.05 to 4°C/minute, especially 0.1 to 2°C/minute. To incorporate an entric polymer material into the ethylcellulose coating walls, the polymer material is added with stirring to the dispersion during the cooling step, particularly at the stage where coating walls of ethylcellulose in the form of "gel" is formed on and around the particles of the core material and the thus-formed coating walls still have fluidity to some extent (i.e. have a viscosity of 0.01 to 5 Pa . s, especially 0.1 to 1 Pa . s). More especially, since the coating walls having a fluidity are formed on and around the core material by cooling the dispersion to about 55 to 75°C, especially about 65°C (while it may somewhat vary depending on the scale of method and cooling rate, etc.), it is preferable to add the enteric polymer material to the dispersion when cooled to said temperature. The enteric polymer material thus added is appropriately penetrated and dispersed into the coating walls. After adding the enteric polymer material, the dispersion is further cooled to a temperature not higher than 40°C (e.g. 30 to 20°C), and thereby, the formed embryonic microcapsules are shrunk and become solid by solvent loss from the coating walls, thus giving stable ethylcellulose microcapsules.

The microcapsules thus obtained may be recovered in conventional manner, such as decantation, centrifugation, filtration and so forth, wherein the microcapsules do not adhere or coagulate each other. The microcapsules thus recovered may, if required, be washed with a solvent such as cyclohexane, petroleum ether, n-hexane, etc. and then dried in conventional manner (e.g. by a hot-air drying method or heat transfer drying method).

The microcapsules of the present invention can be applied to not only pharmaceutically active compounds or medicaments but also other various substances such as veterinary drugs, foodstuffs, or the like. The pharmaceutically active compounds or medicaments, to which the microcapsules of the present

0 077 956

invention and the process for the preparation thereof can be applied, are, for example, vitamines (e.g. ascorbic acid), amino acids (e.g. potassium aspartate, magnesium aspartate), peptides (e.g. insulin), chemotherapeutics (e.g. sulfamethizole) antibiotics (e.g. benzylpenicillin potassium salt), respiratory stimulants (e.g. dimefline hydrochloride), antitussives and expectorants (e.g. tipepidine dibenzoate, bromhexine hydrochloride, trimetoquinol hydrochloride), anti-tumor agents (e.g. 5-fluorouracil, bleomycine hydrochloride), autonomic agents (e.g. N-butylscopolammonium bromide), neuro-pyscotropic agents (e.g. calcium N-(γ,γ-dihydroxy-β,β-dimethylbutyryl)-γ-aminobutyrate), local anesthetics (e.g. oxethazaine), muscle relaxants (e.g. phenprobamate), agents affecting digestive organs (e.g. methyl-methionine sulfonium chloride, 1,1-dimethyl-5-methoxy-3-(dithien-2-ylmethylene)piperidinium bromide, precipitated calcium carbonate, trimebutine maleate), anti-histaminics (e.g. diphenhydramine hydrochloride), antidotes (e.g. D-penicillamine, diferoxamine mesylate), hypnotics and sedatives (e.g. flurazepam hydrochloride), antiepileptics (e.g. sodium valproate), antipyretics, analgesics and antiinflammatory agents (e.g. acetylsalicylic acid, indometacin, naproxen), cardiotonics (e.g. digoxin, proscillaridin), antiarrhythmic agents (e.g. oxprenolol hydrochloride), diuretics (e.g. penfluzide), vasodilators (e.g. diltiazom hydrochloride), antilipaemics (e.g. sodium dextran sulfate), nutrients, tonics and alternatives (e.g. calcium L-aspartate), anticoagulants (e.g. heparin calcium), agents for liver disease (e.g. phosphorylcholine chloride calcium salt), antidiabetic agents (e.g. carbutamide), antihypertensives (e.g. clonidine hydrochloride), or the like. The medicament to be microencapsulated may also be a composition containing such pharmaceutically active compound together with an inert excipient or vehicle.

When the microcapsules containing an enteric polymer material in the coating walls of the present invention are administered, the release of the active compound does not occur in stomach, but the enteric polymer material is easily dissolved in intestinal tract and thereby the microcapsules become porous. Accordingly, the liquid in intestinal tract can easily penetrate into the microcapsules and thereby the release of active compound is promoted. Thus, when pharmaceutical compounds which are decomposed or inactivated by gastric juice or can easily be absorbed in intestinal tract are microencapsulated by the present invention, they are sufficiently protected in stomach and are rapidly released in intestinal tract. Besides, when a water-swellable polymer material is incorporated into the core material, the active compound is likewise protected in stomach, but the enteric polymer material in the ethylcellulose coating walls is rapidly dissolved in intestinal tract to make the coating walls porous, by which the penetration of liquid into the core material is promoted and then the water-swellable polymer material absorbs the liquid and swells. Owing to the swelling pressure, the core material is finely cracked and thereby release of the active compound is further promoted. That is, by coaction of the phenomenon that the coating walls become porous and the capillarity by fine cracking in the core material, the release of the active compound in intestinal tract is promoted. In addition to the above advantages, the microcapsules of the present invention have an appropriately improved ethylcellulose coating walls by incorporating an enteric polymer material into the coating walls, by which the microcapsules show excellent compatibility with various carrier and also excellent free-flowing characteristics and further the microcapsules can easily be tabletted without undesirable sticking or capping. The microcapsules of the present invention show also less unpleasant feeling when administered.

The present invention is illustrated by the following Experiments and Examples, wherein "part" means "part by weight" unless specified otherwise. Throughout the specification and claims, the terms "alkyl" and "alkenyl" should be interpreted as referring to alkyl having one to 8 carbon atoms, preferably one to 4 carbon atoms and alkenyl having 2 to 4 carbon atoms, respectively.

Experiment I

Pyridoxal phosphate-containing microcapsules (an enteric polymer material being incorporated into the coating walls) were prepared according to the following methods. Then, the yield of microcapsules thus obtained, the content of the active ingredient contained in the microcapsules, the release rate (%) of the active ingredient therefrom in the first liquid as defined in the Pharmacopoeia of Japan 10th-Edition, Disintegration Test, and the 50% release time ($T_{50}$) (i.e. a period of time which was necessary to release 50% of the active ingredient from the microcapsules) in the second liquid were examined, respectively.

(i) Core material

To a mixture of pyridoxal phosphate (150 parts) and lactose (129 parts) was added a solution of hydroxypropyl methylcellulose phthalate (21 parts) in 80% aqueous ethanol (30 parts), and the mixture was kneaded in a usual manner to form granules. The granules were dried and regulated to a particle size of 105 to 210 μm.

(ii) Preparation of microcapsules

To cyclohexane (600 ml) were added a silicone resin (conformable to the requirements of 4th Official Compendium of Food Additives in Japan, i.e. a mixture of dimethyl polysiloxane (viscosity: 100 to 1,100 mm$^2$/s at 25°C) and 3 to 15% by weight of silicon dioxide) (18 g) and ethylcellulose (ethoxy content: 48.5%, viscosity: 100 mPa . s) (10 g) and the mixture was dissolved by heating at 80°C. After dispersing a core material (40 g) to the solution, the dispersion was cooled with stirring at 400 r.p.m. When the temperature

4

became to about 65°C, finely divided particles of hydroxypropyl methylcellulose phthalate were added in an amount as shown in the following Table 1 in order to incorporate them into the coating walls and then the mixture was cooled to room temperature. The microcapsules thus formed were separated, washed with n-hexane and dried. Said microcapsules were passed through JIS (Japanese Industrial Standard) standard sieve (350 μm aperture) to give pyridoxal phosphate-containing microcapsules which met the requirements of "powders" specified in the Pharmacopoeia of Japan 10th-Edition.

As a reference, microcapsules were prepared in the same manner as described above except that no hydroxypropyl methylcellulose phthalate was added.

(iii) Results

The results are shown in Table 1. As is clear from the results, when the addition amount of the hydroxypropyl methylcellulose phthalate was larger, the release of the active ingredient in the second liquid was more promoted.

TABLE 1

| Experiment No. | Amount of hydroxypropyl methylcellulose phthalate* (g) | Yield of micro-capsules (g) | Content of active ingredient in capsules (%) | Active in-gredient released after 2 hrs. in the first liquid (%) | $T_{50}$ in the second liquid (minute) |
|---|---|---|---|---|---|
| The present invention | | | | | |
| 1 | 10 | 58 | 33.4 | 9.8 | 68 |
| 2 | 50 | 95 | 21.0 | 8.0 | 36 |
| 3 | 100 | 147 | 13.5 | 10.1 | 10 |
| Reference 4 | — | 49 | 40.8 | 8.4 | 260 |

*) It had methoxy content: 22.2%, hydroxypropoxy content: 7.5%, and carboxybenzoyl content: 21.0%.

Experiment II

Pyridoxal phosphate-containing microcapsules (a water-swellable polymer material being incorporated into the core material, and an enteric polymer material being incorporated into the coating walls) were prepared, and the yield of microcapsules, the content of the active ingredient contained in the microcapsules, the release rate of the active ingredient therefrom in the first liquid and $T_{50}$ in the second liquid were examined, likewise.

(i) Core material

To a mixture of pyridoxal phosphate (150 parts), carboxymethyl cellulose ("a" part) and lactose (129 — "a" parts) was added a solution of a white dextrin (21 parts) in 40% aqueous ethanol (21 parts), and the mixture was kneaded in a usual manner to give granules. The granules were dried and regulated to a particle size of 105 to 210 μm.

(ii) Preparation of microcapsules

To cyclohexane (600 ml) were added dimethyl polysiloxane (viscosity: 1,000,000 mm²/s at 25°C) (18 g) and ethylcellulose (ethoxy content: 47.5%, viscosity: 110 mPa . s) (10 g), and the mixture was dissolved by heating at 80°C. After dispersing a core material (40 g) to the solution, the dispersion was cooled with stirring at 400 r.p.m. When the temperature became to about 65°C, finely divided particles (50 g) of hydroxypropyl methylcellulose (methoxy content: 21.4%, hydroxypropoxy content: 6.7%, carboxybenzoyl content: 28.5%) in order to incorporate them into the coating walls and then the mixture was cooled to room temperature. The microcapsules thus formed were separated, washed with n-hexane and dried. Said microcapsules were passed through JIS standard sieve (350 μm aperture) to give pyridoxal phosphate-containing microcapsules which met the requirements of "powders".

(iii) Results

The results are shown in Table 2.

5

# 0 077 956

TABLE 2

| Experiment No. | Carboxymethyl cellulose ("a" part) | Yield of micro- capsules (g) | Content of active ingredient in capsules (%) | Active in- gredient released after 2 hrs. in the first liquid (%) | $T_{50}$ in the second liquid (minute) |
|---|---|---|---|---|---|
| The present invention 1 | 129 | 98 | 20.1 | 9.8 | 8 |
| 2 | 60 | 96 | 20.7 | 10.1 | 19 |
| 3 | — | 97 | 20.6 | 9.2 | 40 |

Example 1

(1) To a mixture of diltiazem hydrochloride (50) parts and lactose (30 parts) was added a solution of polyvinyl acetate (20 parts) in ethyl acetate (20 parts), and the mixture was kneaded in a usual manner to give granules. The granules were dried and regulated to a particle size of 105 to 210 μm.

(2) To cyclohexane (600 ml) were added the same silicone resin as used in Experiment I (18 g) and the same ethylcellulose as used in Experiment I (15 g), and the mixture was dissolved by heating at 80°C. After dispersing the core material as obtained in the above (1) (15 g) to the solution, the dispersion was cooled with stirring at 400 r.p.m. When the temperature became to about 65°C, the enteric polymer material as shown in Table 3 (45 g) was added in order to incorporate it into the coating walls and then the mixture was cooled to room temperature. The microcapsules thus formed were washed with n-hexane and dried. Said microcapsules were passed through JIS standard sieve (350 μm aperture) to give diltiazem hydrochloride-containing microcapsules which met the requirements of "powders".

6

# 0 077 956

TABLE 3

| Enteric polymer material | Yield of microcapsules (g) | Content of active ingredient in microcapsules (%) |
|---|---|---|
| 1 Methyl acrylate-meth-acrylic acid copolymer (1:1 by mole) | 72 | 10.3 |
| 2 Methyl acrylate-meth-acrylic acid-methyl methacrylate copolymer (1:1.2:1.2 by mole) | 67 | 11.1 |
| 3 Methyl methacrylate-methacrylic acid copolymer (52.4:47.6 by weight) | 70 | 10.7 |
| 4 Methyl methacrylate-methacrylic acid copolymer (70.8:29.2 by weight) | 71 | 10.5 |
| 5 Carboxymethyl-ethyl-cellulose (substitution degree of carboxymethyl: 0.65, substitution degree of ethoxy: 2.1) | 74 | 10.1 |
| 6 Cellulose acetate phtha-late (acetyl content: 20.8%, carboxybenzoyl content: 34.5%) | 70 | 10.7 |
| 7 Shellac | 71 | 10.1 |
| 8 Zein | 69 | 10.7 |
| 9 Methacrylic acid-ethyl acrylate copolymer (1:1 by mole) | 71 | 10.0 |

Example 2

(1) To a mixture of trimethoquinol hydrochloride (chemical name: l-1-(3,4,5-trimethoxybenzyl)-6,7-(dihydroxy-1,2,3,4-tetrahydroisoquinoline hydrochloride) (25 parts), lactose (45 parts), white dextrin (10 parts) and a water-swellable polymer material as shown in Table 4 (20 parts) was added a 40% aqueous ethanol (10 parts), and the mixture was kneaded in a usual manner to give granules. The granules were dried and regulated to a particle size of 105 to 210 µm.

(2) To cyclohexane (600 ml) were added a mixture of two polyisobutylenes (molecular weight: 50,000 and 1,200,000, respectively, 1:1 by weight) (18 g) and the same ethylcellulose as used in Experiment I (12 g), and the mixture was dissolved by heating at 80°C. After dispersing the core material as obtained in the above (1) (48 g) to the solution, the dispersion was cooled with stirring at 400 r.p.m. When the temperature became to about 65°C, cellulose acetate phthalate (acetyl content: 20.8%, carboxybenzoyl content: 34.5%) (60 g) was added in order to incorporate it into the coating walls and then the mixture was cooled to room temperature. The microcapsules thus formed were separated washed with n-hexane and dried. Said microcapsules were treated in the same manner as described in Example 1 to give trimethoquinol hydrochloride-containing microcapsules.

7

TABLE 4

| Water-swellable polymer material | Degree of swelling | Yield of microcapsules (g) | Content of active ingredient (%) |
|---|---|---|---|
| Agar-agar | 4.0 | 115 | 10.3 |
| Carboxymethylcellulose calcium | 2.1 | 117 | 10.2 |
| Pectinic acid | 3.5 | 114 | 10.5 |
| Gum arabic crosslinked with epichlorohydrin | 8.5 | 118 | 10.1 |
| Dextran crosslinked with epichlorohydrin | 15 | 109 | 10.9 |
| Divinylbenzeneacrylic acid copolymer | 4.4 | 113 | 10.6 |
| Self-crosslinked polyacrylic acid | 20 | 108 | 11.1 |
| Potato starch | 1.4 | 117 | 10.1 |
| Carboxymethylcellulose | 2.3 | 110 | 10.9 |
| Crystalline cellulose | 1.2 | 115 | 10.3 |

Example 3

To cyclohexane (600 ml) were added polyethylene (molecular weight: 7,000) (18 g) and the same ethylcellulose as used in Experiment I (12 g) and the mixture was dissolved by heating at 80°C. After dispersing 1-methyl-5-methoxy-3-(dithien-2-ylmethylene)piperidium hydrobromide (I) (particle size: 105—210 μm) (48 g) to the solution, the dispersion was cooled with stirring at 400 r.p.m. When the temperature became to about 65°C, carboxymethyl ethylcellulose (substitution degree of carboxymethyl: 0.55, substitution degree of ethoxy: 2.2) (60 g) was added in order to incorporate it into the coating walls, and the mixture was cooled to room temperature. The microcapsules thus formed were treated in the same manner as described in Example 2-(2) to give the active compound (I)-containing microcapsules (116 g) which met the requirements of "powders" and had a content of the active compound (I) of 40.1%.

**Claims**

1. A method of preparing a core material-containing microcapsules having a core material coated with a mixture of ethylcellulose and an enteric polymer material, characterized by the following steps:
(a) ethylcellulose is dissolved in a solvent at a temperature of about 75—80°C,
(b) particles of a core material are dispersed in the solution thus obtained,
(c) the dispersion is cooled until coating walls having a viscosity of 0.01 to 5 Pa . s are formed on and around particles of the core material,
(d) an enteric polymer material is added to the dispersion,
(e) the dispersion containing the enteric polymer material is further cooled to a temperature not higher than 40°C until the resultant embryonic microcapsules shrink and become solid by solvent loss from the coating walls, and
(f) the thus-formed microcapsules are recovered therefrom.

2. The method according to claim 1, wherein the enteric polymer material is a member selected from the group consisting of an organic dicarboxylic acid ester of a hydroxyalkyl alkylcellulose or cellulose acetate, a carboxyalkyl alkylcellulose, a copolymer of an alkenylcarboxylic acid and an alkyl ester of an alkenylcarboxylic acid, a copolymer of an alkenylcarboxylic acid and two alkyl esters of an alkenylcarboxylic acid, zein and shellac.

3. The method according to claim 1, wherein the enteric polymer material is a member selected from the group consisting of a phthalic acid ester of a hydroxyalkyl alkylcellulose or cellulose acetate, a carboxyalkyl alkylcellulose, a copolymer of methacrylic acid and an alkyl acrylate, a copolymer of methacrylic acid, an alkyl acrylate and an alkyl methacrylate, zein and shellac.

4. The method according to claim 1, wherein the enteric polymer material is a member selected from the group consisting of cellulose acetate phthalate, hydroxypropyl methylcellulose phthalate, carboxymethyl ethylcellulose, copolymer of methacrylic acid and methyl acrylate, copolymer of

8

methacrylic acid and ethyl acrylate, copolymer of methacrylic acid and methyl methacrylate, copolymer of methacrylic acid, methyl acrylate and methyl methacrylate, zein and shellac.

5. The method according to any one of claims 1 through 4, wherein ethylcellulose having an ethoxy content of 46.5 to 55 W/W % is used.

6. The method according to any one of claims 1 through 4, wherein ethylcellulose having an ethoxy content of 46.5 to 55 W/W % and a viscosity [measured at 25°C with respect to a 5 W/W % solution in toluene-ethanol (4:1)] of 3 to 500 mPa . s (3 to 500 cP) is used.

7. The method according to claim 5, wherein the solvent is cyclohexane.

8. The method according to claim 7, wherein the ethylcellulose is used in an amount of 0.01 to 10 grams per gram of core material.

9. The method according to claim 7, wherein the enteric polymer material is used in an amount of at least 0.01 gram per gram of ethylcellulose.

10. The method according to claim 7, wherein the enteric polymer material is used in an amount of 0.05 to 20 grams per gram of ethylcellulose.

11. The method according to claim 7, wherein particles of the core material having 30 to 1,000 µm are dispersed in the solution of ethylcellulose, and the enteric polymer material having a particle size not more than 300 µm is added at the stage when the coating walls have a viscosity of 0.01 to 5 Pa . s (0.1 to 50 P).

12. The method according to claim 11, wherein particles of the core material contain a water-swellable polymer material.

13. The method according to claim 12, wherein the water-swellable polymer material is a member selected from the group consisting of agar-agar, pectinic acid, alginic acid, cellulose, starch, a carboxyalkyl cellulose, a carboxyalkyl cellulose calcium, a copolymer of divinylbenzene and an alkenylcarboxylic acid, a cross-linked gum arabic, a cross-linked dextran, and a cross-linked polyalkenylcarboxylic acid.

14. The method according to claim 12, wherein the water-swellable polymer material is a member selected from the group consisting of agar-agar, pectinic acid, alginic acid, cellulose, starch, carboxymethyl cellulose, carboxymethyl cellulose calcium, copolymer of divinylbenzene and acrylic acid, gum arabic cross-linked with epichlorohydrin, dextran cross-linked with epichlorohydrin, or self-crosslinked polyacrylic acid.

15. The method according to any one of claims 11 through 14, wherein the dispersion is cooled at a rate of 0.05 to 4°C/minute, and the enteric polymer material is added to the dispersion at a temperature of about 55 to 75°C.

16. The method according to any one of claims 11 through 14, wherein at least one member selected from a phase-separation-inducing agent, an organosilicon polymer compound and a surfactant is further added to the solution of ethylcellulose.

## Patentansprüche

1. Verfahren zur Herstellung von ein Kernmaterial enthaltenden Mikrokapseln mit einem Kernmaterial, das mit einer Mischung aus Ethylcellulose und einem enterischen Polymermaterial beschichtet ist, gekennzeichnet durch die folgenden Stufen:

(a) Ethylcellulose wird in einem Lösungsmittel bei einer Temperatur von etwa 75 bis 80°C gelöst,

(b) Teilchen eines Kernmaterials werden in der so erhaltenen Lösung dispergiert,

(c) die Dispersion wird gekühlt, bis sich eine Überzugswand mit einer Viskosität von 0,01 bis 5 Pa . s auf und um die Teilchen des Kernmaterials ausgebildet hat,

(d) ein enterisches Polymermaterial wird zu der Dispersion gegeben,

(e) die das enterische Polymermaterial enthaltende Dispersion wird weiter auf eine Temperatur von nicht mehr als 40°C gekühlt, bis die gebildeten embryonischen Mikrokapseln schrumpfen und durch Lösungsmittelverlust aus der Beschichtungswand fest werden, und

(f) die so gebildeten Mikrokapseln werden gewonnen.

2. Verfahren gemäss Anspruch 1, worin das enterische Polymermaterial ausgewählt ist aus der Gruppe bestehend aus einem organischen Dicarbonsäureester einer Hydroxyalkylalkylcellulose oder Celluloseacetat, einer Carboxyalkylalkylcellulose, einem Copolymer aus einer Alkenylcarbonsäure und einem Alkylester einer Alkenylcarbonsäure, einem Copolymer aus einer Alkenylcarbonsäure und zwei Alkylestern von Alkenylcarbonsäuren, Zein und Schellack.

3. Verfahren gemäss Anspruch 1, worin das enterische Polymermaterial ausgewählt ist aus der Gruppe bestehend aus einem Phthalsäureester von Hydroxyalkylalkylcellulose oder Celluloseacetat, einer Carboxyalkylalkylcellulose, einem Copolymer aus Methacrylsäure und einem Alkylacrylat, einem Copolymer aus Methacrylsäure, einem Alkylacrylat und einem Alkylmethacrylat, Zein und Schellack.

4. Verfahren gemäss Anspruch 1, worin das enterische Polymermaterial ausgewählt ist aus der Gruppe bestehend aus Celluloseacetatphthalat, Hydroxypropylmethylcellulosephthalat, Carboxymethylethylcellulose, einem Copolymer von Methacrylsäure und Methylacrylat, einem Copolymer von Methacrylsäure und Ethylacrylat, einem Copolymer von Methacrylsäure und Methylmethacrylat, einem Copolymer von Methacrylsäure, Methylacrylat und Methylmethacrylat, Zein und Schellack.

5. Verfahren gemäss einem der Ansprüche 1 bis 4, worin Ethylcellulose mit einem Ethoxygehalt von 46,5 bis 55 W/W-% verwendet wird.

# 0 077 956

6. Verfahren gemäss einem der Ansprüche 1 bis 4, worin Ethylcellulose mit einem Ethoxygehalt von 46,5 bis 55 W/W-% und mit einer Viskosität (gemessen bei 25°C bei einer 5 W/W-% Lösung in Toluol-Ethanol — 4:1) von 3 bis 500 mPa . s (3 bis 500 cP) verwendet wird.

7. Verfahren gemäss Anspruch 5, worin das Lösu:   mittel Cyclohexan ist.

8. Verfahren gemäss Anspruch 7, worin die Ethylcellulose in einer Menge von 0,01 bis 10 g pro g des Kernmaterials verwendet wird.

9. Verfahren gemäss Anspruch 7, worin das enterische Polymermaterial in einer Menge von wenigstens 0,01 g pro g Ethylcellulose verwendet wird.

10. Verfahren gemäss Anspruch 7, worin das enterische Polymermaterial in einer Menge von 0,05 bis 20 g pro g Ethylcellulose verwendet wird.

11. Verfahren gemäss Anspruch 7, worin Teilchen des Kernmaterials von 30 bis 1.000 µm in einer Lösung von Ethylcellulose dispergiert werden und das enterische Polymermaterial mit einer Teilchengrösse von nicht mehr als 300 µm in dem Stadium zugegeben wird, in dem die Überzugswände eine Viskosität von 0,01 bis 5 Pa . s (0,1 bis 50 P) haben.

12. Verfahren gemäss Anspruch 11, worin die Teilchen des Kernmaterials ein wasserquellbares Polymermaterial enthalten.

13. Verfahren gemäss Anspruch 12, worin das wasserquellbare Polymermaterial ausgewählt ist aus der Gruppe bestehend aus Agar-Agar, Pectinsäure, Alginsäure, Cellulose, Stärke, einer Carboxyalkyl-cellulose, einem Carboxyalkylcellulosecalcium, einem Copolymer aus Divinylbenzol und einer Alkenylcarbonsäure, einem vernetzten Gummiarabikum, einem vernetzten Dextran und einer vernetzten Polyalkenylcarbonsäure.

14. Verfahren gemäss Anspruch 12, worin das wasserquellbare Polymermaterial ausgewählt ist aus der Gruppe bestehend aus Agar-Agar, Pectinsäure, Alginsäure, Cellulose, Stärke, Carboxymethylcellulose, Carboxymethylcellulosecalcium, einem Copolymer von Divinylbenzol und Acrylsäure, Gummiarabikum, vernetzt mit Epichlorhydrin, Dextran, vernetzt mit Epichlorhydrin, oder selbstvernetzter Polyacrylsäure.

15. Verfahren gemäss einem der Ansprüche 11 bis 14, worin die Dispersion in einer Rate von 0,05 bis 4°C/min gekühlt wird und das enterische Polymermaterial zur Dispersion bei einer Temperatur von etwa 55 bis 75°C zugegeben wird.

16. Verfahren gemäss einem der Ansprüche 11 bis 14, worin wenigstens ein Glied, ausgewählt aus einem die Phasentrennung einleitenden Mittel, einer Organosilicium-Polymerverbindung und einer oberflächenaktiven Verbindung, zusätzlich zu der Ethylcelluloselösung gegeben wird.


## Revendications

1. Procédé pour préparer des microcapsules contenant une matière de noyau revêtue par un mélange d'éthylcellulose et d'une matière polymère entérique, caractérisé par les stades opératoires suivants:

(a) on dissout de l'éthylcellulose dans un solvant à une température d'environ 75—80°C,

(b) on disperse des particules d'une matière de noyau dans la solution ainsi obtenue,

(c) on refroidit la dispersion jusqu'à formation de parois de revêtement ayant une viscosité de 0,01 à 5 Pa . s sur les particules de la matière de noyau et autour de ces particules,

(d) on ajoute une matière polymère entérique à la dispersion,

(e) on refroidit plus profondément la dispersion contenant la matière polymère entérique jusqu'à une température ne dépassant pas 40°C jusqu'à ce que les microcapsules embryonnaires formées se rétractent et se solidifient par perte de solvant des parois de revêtement, et

(f) on recueille les microcapsules ainsi formées.

2. Procédé selon la revendication 1, dans lequel la matière polymère entérique est choisie dans le groupe consistant en un ester d'acide organique dicarboxylique d'une hydroxyalkyl-alkylcellulose ou de l'acétate de cellulose, une carboxyalkyl-alkylcellulose, un copolymère d'un acide alcénylcarboxylique et d'un ester alkylique d'un acide alcénylcarboxylique, un copolymère d'un acide alcénylcarboxylique et de deux esters alkyliques d'un acide alcénylcarboxylique, la zéine et la gomme-laque.

3. Procédé selon la revendication 1, dans lequel la matière polymère entérique est choisie dans le groupe consistant en un ester phtalique d'une hydroxyalkyl-alkylcellulose ou de l'acétate de cellulose, une carboxyalkyl-alkylcellulose, un copolymère de l'acide méthacrylique et un acrylate d'alkyle, un copolymère de l'acide méthacrylique, d'une acrylate d'alkyle et d'un méthacrylate d'alkyle, la zéine et la gomme-laque.

4. Procédé selon la revendication 1, dans lequel la matière polymère entérique est choisie dans le groupe consistant en l'acétate-phtalate de cellulose, le phtalate d'hydroxypropyl-méthylcellulose, le carboxyméthyl-éthylcellulose, un copolymère de l'acide méthacrylique et de l'acrylate de méthyle, un copolymère de l'acide méthacrylique et de l'acrylate d'éthyle, un copolymère de l'acide méthacrylique et du méthacrylate de méthyle, un copolymère de l'acide méthacrylique, de l'acrylate de méthyle et du méthacrylate de méthyle, la zéine et la gomme-laque.

5. Procédé selon l'une quelconque des revendications 1 à 4, dans lequel on utilise de l'éthylcellulose contenant de 46,5 à 55% en poids de groupes éthoxy.

6. Procédé selon l'une quelconque des revendications 1 à 4, dans lequel on utilise de l'éthylcellulose ayant une teneur en groupes éthoxy de 46,5 à 55% en poids et une viscosité (mesurée à 25°C sur une solution à 5% en poids dans un mélange toluène-éthanol, 4:1) de 3 à 500 mPa . s (3 à 500 cP).

7. Procédé selon la revendication 5, dans lequel le solvant est le cyclohexane.

8. Procédé selon la revendication 7, dans lequel l'éthylcellulose est utilisée en proportion de 0,01 à 10 g par gramme de la matière de noyau.

9. Le procédé selon la revendication 7, dans lequel la matière polymère entérique est utilisée en proportion d'au moins 0,01 g par gramme d'éthylcellulose.

10. Le procédé selon la revendication 7, dans lequel la matière polymère entérique est utilisée en proportion de 0,05 à 20 g par gramme d'éthylcellulose.

11. Le procédé selon la revendication 7, dans lequel on disperse des particules de la matière de noyau à une dimension de 30 à 1 000 µm dans la solution d'éthylcellulose, et on ajoute la matière polymère entérique à une dimension de particules ne dépassant pas 300 µm au moment où les parois de revêtement ont une viscosité de 0,01 à 5 Pa . s (0,1 à 50 P).

12. Le procédé selon la revendication 11, dans lequel les particules de la matière de noyau contiennent une matière polymère gonflable à l'eau.

13. Le procédé selon la revendication 12, dans lequel la matière polymère gonflable à l'eau est choisie dans le groupe consistant en la gélose, l'acide pectinique, l'acide alginique, la cellulose, l'amidon, une carboxyalkyl-cellulose, une carboxyalkyl-cellulose calcique, un copolymère de divinylbenzène et d'un acide alcénylcarboxylique, une gomme arabique réticulée, un dextrane réticulé, et un acide polyalcényl-carboxylique réticulé.

14. Le procédé selon la revendication 12, dans lequel la matière polymère gonflable à l'eau est choisie dans le groupe consistant en la gélose, l'acide pectinique, l'acide alginique, la cellulose, l'amidon, la carboxyméthyl-cellulose, la carboxyméthyl-cellulose, calcique, un copolymère du divinylbenzène et de l'acide acrylique, la gomme arabique réticulée par l'épichlorhydrine, le dextrane réticulé par l'épichlorhydrine, ou l'acide polyacrylique auto-réticulé.

15. Le procédé selon l'une quelconque des revendications 11 à 14, dans lequel la dispersion est refroidie à une vitesse de 0,05 à 4°C/min, et la matière polymère entérique est ajoutée à la dispersion à une température d'environ 55 à 75°C.

16. Le procédé selon l'une quelconque des revendications 11 à 14, dans lequel on ajoute à la solution d'éthylcellulose au moins un composé choisi parmi les agents inducteurs de séparation de phase, les composés polymères organosiliciés et les agents tensioactifs.